(19) Europäisches Patentamt
European Patent Office
Office européen des brevets

(11) Veröffentlichungsnummer: **0 156 280**
**B1**

(12) # EUROPÄISCHE PATENTSCHRIFT

(45) Veröffentlichungstag der Patentschrift: **07.02.90**

(51) Int. Cl. 5: **C 07 K 7/06**, C 07 K 7/20, C 07 K 5/08, A 61 K 37/02

(21) Anmeldenummer: **85103106.2**

(22) Anmeldetag: **18.03.85**

(54) **Verfahren zur racematarmen Herstellung von Peptidzwischenprodukten der Gonadorelin- und Gonadorelinanaloga-Synthese und neue Zwischenprodukte bei diesem Verfahren.**

(30) Priorität: **27.03.84 DE 3411224**

(43) Veröffentlichungstag der Anmeldung: **02.10.85 Patentblatt 85/40**

(45) Bekanntmachung des Hinweises auf die Patenterteilung: **07.02.90 Patentblatt 90/06**

(84) Bennante Vertragsstaaten: **AT BE CH DE FR GB IT LI LU NL SE**

(56) Entgegenhaltungen:
**EP-A-0 041 243**
**EP-A-0 056 274**
**US-A-3 915 947**
**US-A-3 963 691**
**US-A-4 086 219**
**US-A-4 101 537**
**US-A-4 272 432**

**JOURNAL OF MEDICINAL CHEMISTRY, Band 22, Nr. 8, 1979, Seiten 935-943, American Chemical Society; G.W. MOERSCH et al.: "Synthesis and biological activity of peptide antagonists of Luliberin (Luteinizing hormone-releasing hormone)"**

(73) Patentinhaber: **HOECHST AKTIENGESELLSCHAFT**
**Postfach 80 03 20**
**D-6230 Frankfurt am Main 80 (DE)**

(72) Erfinder: **Uhmann, Rainer, Dr.**
**An der Landwehr 5**
**D-6239 Kriftel (DE)**
Erfinder: **Radscheit, Kurt, Dr.**
**Johann-Strauss-Strasse 20**
**D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 156 280 B1

LIBERGRAF, STOCKHOLM 1990

**Beschreibung**

Die Erfindung betrifft ein Verfahren zur Herstellung von Peptiden der Formel I

$$U - A^1 - A^2 - A^3 - A^4 - A^5 - X \quad (I)$$

in welcher
U eine Urethanschutzgruppe,
$A^1$ Trp oder D-Trp,
$A^2$ Ser, Ala oder Thr,
$A^3$ Tyr oder Phe,
$A^4$ Gly, den Rest einer D-Aminosäure oder den Rest eines D-Aminosäurederivates,
$A^5$ Leu, N-Methyl-Leu, N-Ethyl-Leu, Ser($Bu^t$), Cys($Bu^t$), Asp($OBu^t$), Glu($OBu^t$), Orn(Boc) oder Lys(Boc) und
X $OBu^t$ oder $A^6$-Pro-Y bedeuten,
wobei
$A^6$ für Arg, Orn, Lys oder Homoarginin und
Y für Gly-$NH_2$, NH-NH-CO-$NH_2$, ($C_1$-$C_3$)-Alkylamino, Cyclopropylamino, mit Hydroxy oder Fluor substituiertes ($C_1$-$C_3$)-Alkylamino oder mit Hydroxy oder Fluor substituiertes Cycloalkylamino steht, das dadurch gekennzeichnet ist, daß man ein Tripeptid der Formel II

$$U - A^1 - A^2 - A^3 - OH \quad (II)$$

in welcher U, $A^1$, $A^2$ und $A^3$ die oben genannte Bedeutung haben, mit einem Peptid der Formel III

$$H - A^4 - A^5 - X \quad (III)$$

in welcher $A^4$, $A^5$ und X die oben genannte Bedeutung haben, oder dessen Derivat umsetzt.

Peptide der allgemeinen Formel I sind ihrerseits Zwischenprodukte bei der Synthese von Gonadorelin (Biochem. Biophys. Res. Commun. 43 [1971] 1334 - 39) oder dessen Analoga, wie sie beispielsweise aus dem US-Patent 4 024 248 bekannt sind.

Gonadorelin (LHRH) ist bekanntlich ein Hormon aus dem Hypothalamus der Formel IV

$$\text{Pyr-His-Trp-Ser-Tyr-Gly-Leu-Arg-Pro-Gly-NH}_2 \quad (IV)$$

das in der Hypophyse die gonadotropen Hormone LH und FSH ausschüttet. Als Gonadorelin (LHRH)-Analoga kommen Peptide in Betracht, in denen einzelne oder mehrere Aminosäuren des LHRH ausgetauscht sind und/oder die Peptidkette durch Verkürzung, Verlängerung und/oder Derivatisierung variiert ist. Von besonderer Bedeutung sind dabei Substitutionen von Glycin in Position 6 durch D-Aminosäuren und in Position 10 durch Gruppen wie Alkylamino. Erwähnt sei z. B. Buserelin (Formel V):

$$\text{Pyr-His-Trp-Ser-Tyr-D-Ser(Bu}^t\text{)-Leu-Arg-Pro-NHEt} \quad (V)$$

Aus der Literatur sind Verfahren zur Herstellung von Verbindungen der Formel I bekannt (Biochem. Biophys. Res. Commun. 45 [1971] 767; US-Patent 4 024 248), bei welchen die Synthese stets nach der Azidmethode erfolgt, die als racemisierungsarmes Verfahren bei derartigen Fragmentsynthesen bekannt ist (Lit.: J. Chem. Soc. [London] 1960, 3902; Helv. Chim. Acta, 44 (1961) 1991).

Dennoch ist bei der Anwendung der Azidsynthese mit zahlreichen Nebenreaktionen zu rechnen. So können 1,2-Diacyl-hydrazine entstehen, vor allem dann, wenn Säureunterschuß während der Azidbildung vorherrscht.

Bei Tyrosin im vorliegenden Fall kann Nitrierung in o-Stellung zur phenolischen Hydroxygruppe und bei Tryptophan kann N-Nitrosierung erfolgen. Auch die Umwandlung von Peptidaziden in die entsprechenden Amide wurde beobachtet (zusammenfassende Lit.: Liebigs Ann., 659 (1962) 168, Synthesis (1974) 549).

Ein gravierender Mangel ist die Instabilität der Peptidazide, die sich schon bei leicht erhöhter Temperatur nach einem Curtius-Abbau in die Isocyanate umlagern können und in der Folge Harnstoffe und andere Nebenprodukte bilden (Helv. Chim. Acta, 44 (1961) 1991, dort S. 1997, Synthesis (1974) 549).

Bei der Nacharbeitung konnte ebenfalls diese Nebenreaktion unter Bildung des Isocyanats und Weiterreaktion zum Harnstoff beobachtet werden (s. Beispiel 4.1 und 4.2). Das Nebenprodukt (Harnstoffderivat) ist sehr schwer vom entsprechenden Peptid abzutrennen und nur mit Hilfe der HPLC nachzuweisen. Das Ausmaß dieser Nebenreaktion kann bis zu 15 % betragen (s. Beispiel 4.2).

Die Ausbeuten der Azidsynthesen sind selten höher als 70 - 80 % d.Th. Ursache dafür sind die erwähnten Nebenreaktionen.

Das Azidverfahren ist unter dem Gesichtspunkt des Produktionsmaßstabs unbrauchbar. Die wehrend des Verfahrens auftretende Stickstoffwasserstoffsäure oder ihre Salze stellen ab einer bestimmten Menge ein technisch schwer zu bewältigendes Sicherheitsrisiko dar. So tritt z. B. beim Umsatz von ca. 25 kg Tripeptidazid im Laufe der Aufarbeitung, beispielsweise bei einer sauren Extraktion mind. 1 kg Stickstoffwasserstoffsäure, die zu heftigen Explosionen führen kann (Lit.: Römpps Chemie Lexikon, 7. Auflage, Frankh'sche Verlagshandlung, Stuttgart 1975, S. 3348).

Um dieses Risiko zu beherrschen, müßten zahlreiche teure meßtechnische Inprozeßkontrollen entwickelt und installiert werden. Außerdem müßten Mutterlaugen und wäßrige Extraktionslösungen vor ihrer Entsorgung von Stickstoffwasserstoffsäure und ihren Salzen befreit werden, was ebenfalls eines größeren analytischen Aufwandes bedürfte.

Die Stickstoffwasserstoffsäure ist außerdem noch unter dem Aspekt der Arbeitshygiene als bedenklich anzusehen; dies drückt sich in einem sehr niedrigen MAK-Wert aus. Der MAK-Wert für $HN_3$ beträgt 0,1 ml/$m^3$ bzw. 0,27 mg/$m^3$ (TRgA 900, 1982).

Diese genannten Gründe verbieten es, das Azidverfahren über einen Labormaßstab hinaus zu vergrößern.

Es wurde nun gefunden, daß man ausgehend von dem auf racemisierungsarmem Wege hergestellten geschützten Tripeptid der Formel II mit Hilfe der fünf unten genannten Kupplungsverfahren überraschenderweise praktisch zu racematfreien Produkten der Formel I kommt.

Das erfindungsgemäße Verfahren z. B. unter der Verwendung der PPA-Methode unterliegt im Gegensatz zum Azidverfahren keinen sicherheitstechnischen Beschränkungen. Darüberhinaus erwies sich das Verfahren vom praktisch-chemischen Standpunkt aus ebenfalls als problemlos. Der Übergang in den Produktionsmaßstab führte weder zum Verlust an Ausbeute noch an Reinheit der Produkte gegenüber dem Labormaßstab (Beispiel 6.1).

Die Umsetzung des Tripeptids der Formel II mit dem entsprechenden Peptid der Formel III mit freier Aminogruppe und geschützter Carboxylgruppe wird vorzugsweise in einem in der Peptidchemie üblichen Lösungsmittel oder auch in Wasser/Lösungsmittel-Gemischen in Gegenwart eines geeigneten Kondensationsmittels durchgeführt, wie z. B.

1. Dicyclohexylcarbodiimid unter Zusatz von 1-Hydroxybenzotriazol (DCCI/HOBt-Methode; Lit.: Chem.Ber. 103 (1970) 788)
2. Dicyclohexylcarbodiimid unter Zusatz von 3-Hydroxy-4-oxo-3,4-dihydro-1,2,3-benzotriazin (DCCI/HOOBt-Methode; Lit.: Chem.Ber.103 (1970) 2034)
3. Dicyclohexylcarbodiimid unter Zusatz von N-Hydroxysuccinimiid (DCCI/HONSu-Methode; Lit.: Z. Naturforsch. 21b (1966) 426)
4. Alkanphosphonsäureanhydrid, wie n-Propylphosphonsäureanhydrid (PPA-Methode; Lit.: Angew. Chemie, Int. Ed. 19 (1980) 133)
5. Dialkylphosphinsäureanhydrid, wie Methylethylphosphinsäureanhydrid (MEPA-Methode; Lit.: US-Patent 4 426 325)

Wenn im zu synthetisierenden Gonadorelinanalogen die Aminosäure in Position 6 ein säurelabiles Aminosäurederivat (wie z. B. D-Ser($Bu^t$), D-Glu($OBu^t$), L-Lys(Boc) usw.) sein soll, ist das Zwischenprodukt der Formel I mit X = $OBu^t$ nicht sinnvoll; der Syntheseweg muß dann über Zwischenprodukte der Formel I mit X $\neq$ $OBu^t$ verlaufen.

Als geeignete Lösungsmittel verwendet man bei dem erfindungsgemäßen Verfahren aus Gründen der Löslichkeit meist polare Lösungsmittel wie z. B. Dimethylacetamid, Dimethylformamid, Dimethylsulfoxid, Phosphorsäure-tris-(dimethylamid), N-Methylpyrrolidon, Wasser oder Mischungen der genannten Lösungsmittel mit Wasser. Letzteres trifft vor allem beim MEPA-Verfahren zu. Aber auch Chloroform, Methylenchlorid oder Essigester kommen zum Einsatz. Die Synthese kann zwischen - 10°C und Raumtemperatur durchgeführt werden. Vorzugsweise beginnt man bei ca. 0°C und erhöht später auf Raumtemperatur.

Eine bevorzugte Verfahrensvariante ist dadurch gekennzeichnet, daß

$A^1$ Trp, $A^2$ Ser, $A^3$ Tyr, $A^4$ Gly, D-Ala, D-Leu, D-Ser($Bu^t$), D-Trp, D-Phe, D-Gln(cyclohexyl), D-Naphthylalanin, D-Benzylhistidin, D-Thr($Bu^t$), D-Cys($Bu^t$), D-Asp($OBu^t$), D-Glu($OBu^t$), D-Orn(Boc) oder D-Lys(Boc), $A^5$ Leu und X $OBu^t$, Arg-Pro-Gly-$NH_2$ oder Arg-Pro-NH-NH-CO-NH bedeuten.

Besonders bevorzugt sind Verfahren zur Herstellung von U-Trp-Ser-Tyr-Gly-Leu-Arg-Pro-Gly-$NH_2$ und U-Trp-Ser-Tyr-D-Ser($Bu^t$)-Leu-Arg-Pro-$NHC_2H_5$, worin U wie oben *definiert* ist.

Bevorzugt sind solche Urethanschutzgruppen U (vgl. Kontakte Merck 3/79, Seite 14, 16), die hydrogenolytisch abspaltbar sind, wie Z, Z($NO_2$), Pyoc, Z($Hal_n$). Dobz oder Moc, insbesondere aber Z.

Der überraschend geringe Racemisierungsgrad bei Anwendung des erfindungsgemäßen Verfahrens wurde mit Hilfe der Hochdruckflüssigkeitschromatographie (HPLC) an den ungeschützten Peptiden der Formel I (U = Wasserstoff) belegt. Stellte man ein entsprechendes Hepta- oder Octapeptid der Formel I wie oben beschrieben her, das in der Position $A^3$ statt Tyr D-Tyr eingebaut hatte, so erhielt man das Produkt, das im Falle einer Racemisierung der Verbindung mit $A^3$ = Tyr während der Kupplung auftreten muß. Da mittels HPLC diese beiden Diastereomeren als ungeschützte Peptide getrennt werden können, konnte man so das Ausmaß der Racemisierung bei den einzelnen Kupplungsmethoden erfassen.

**Tab. I Racemisierungsgrad - Beispiel:**

Z-Trp-Ser-Tyr-D-Ser($Bu^t$)-Leu-Arg-Pro-NHEt; die Synthese der genannten Verbindungen wird nach den verschiedenen Methoden durchgeführt, das Reaktionsprodukt wird jeweils ohne Zwischenreinigung der Hydrierung unterworfen und als H-Trp-Ser-Tyr-D-Ser($Bu^t$)-Leu-Arg-Pro-NHEt mittels HPLC charakterisiert.

HPLC-Methode:

Säule: Nucleosil(R) 5SA (4 x 300 mm); Elutionsmittel: 40 % Puffer / 60 % Acetonitril (Puffer: 1 %-ig $KH_2PO_4$; pH: 5,8), Fluß: 1 ml/min; Detektor: UV - 220 nm.

| Synthesemethode | % Anteil des D-Isomeren $^{+)}$ |
|---|---|
| DCCI/HOBt | 3 - 5 |
| DCCI/HOOBt | 1,5 - 2,5 |
| DCCI/HONSu | 1,5 - 2,5 |
| PPA | 1 - 2 |
| MEPA | 1,5 - 2,5 |
| Azid | 1 - 2 |

$^{+)}$ Die Retentionszeit des D-Isomeren H-Trp-Ser-D-Tyr-D-Ser($Bu^t$)-Leu-Arg-Pro-NHEt wurde nach gezielter Herstellung der Verbindung mittels der PPA-Methode ermittelt.

Bemerkenswert ist, daß neben dem geringen Umfang an Racemisierung bei Anwendung der PPA-Methode, die Ausbeute und die Reinheit der Produkte besonders hoch ist.

**Tab. II Vergleich: Reinheit/Ausbeute**

Beispiel: Z-Trp-Ser-Tyr-D-Ser($Bu^t$)-Leu-Arg-Pro-NHEt; das Reaktionsprodukt wird nach Isolierung gewogen, daraus die theoretische Ausbeute errechnet und mit Hilfe der HPLC wird ein relatives Reinheitskriterium erstellt.
HPLC-Methode:

Säule: Nucleosil$^{(R)}$ 5SA (4 x 300 mm); Elutionsmittel: 40 % Puffer / 60 % Acetonitril (Puffer: 1 %-ig $KH_2PO_4$; pH: 5,8); Fluß: 1 ml/min Detektor: UV- 220 nm.

| Synthesemethode | Ausbeute (% d.Th.) | Reinheit; Flächen-% $^{+)}$ |
|---|---|---|
| DCCI/HOBt | 69 | 73 |
| DCCI/HOOBt | 72 | 75 |
| DCCI/HONSu | 75 | 77 |
| PPA | 85 - 88 | 80 |
| MEPA | 63 | 76 |
| Azid | 70 - 80 | 55 |

$^{+)}$ Relatives Reinheitskriterium nach HPLC; die Summe der Peak-Flächen aller UV-absorbierenden Substanzen (bei 220 nm) des Reaktionsproduktchromatogramms wird gleich 100 gesetzt. x Flächen-% entfallen auf die gewünschte Substanz (100 %-Methode).

Die Erfindung betrifft weiterhin Tripeptide der Formel II

$$U - A^1 - A^2 - A^3 - OH \qquad (II)$$

in der U, $A^1$, A2 und $A^3$ wie oben definiert sind, insbesondere

Z - Trp - Ser - Tyr - OH,

sowie ein Verfahren zur Herstellung dieser Tripeptide durch in der Peptidchemie übliche Fragmentkondensation von Peptidbruchstücken nach dem Kondensationsschema (1 - 2) + 3 oder 1 + (2 - 3), wobei andere funktionelle Gruppen gegebenenfalls durch im alkalischen oder sauren Medium abspaltbare Schutzgruppen gegebenenfalls intermediär blockiert werden.

Die Synthese der Tripeptide der Formel II erfolgt vorzugsweise auf racemisierungsarmem Wege, d.h. unter Vermeidung von Synthesestufen, die eine Verseifungsreaktion beinhalten, wie beispielsweise die im folgenden Schema beschriebene Synthese von Z-Trp-Ser-Tyr-OH.

**Schema:**

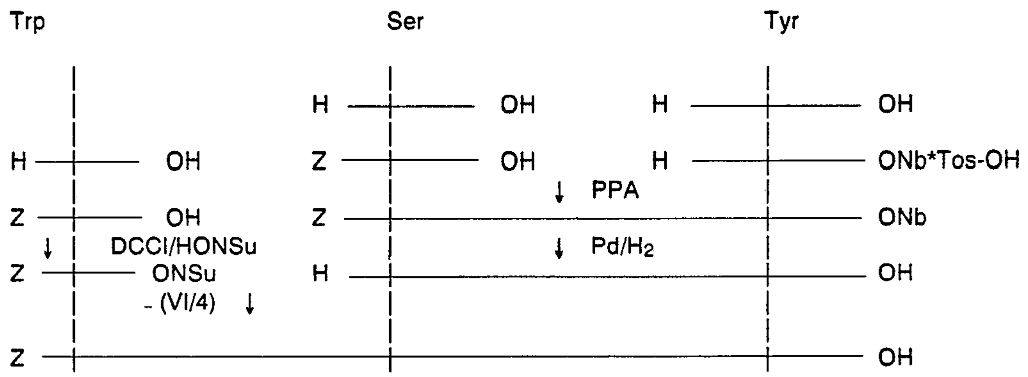


(VI/I)

(VI/2)

(VI/3)

(II)

Die Verbindungen II, VI/2 und VI/3 sind in der Literatur nicht beschrieben. Verbindung VI/1 wurde nach Shields, McGregor und Carpenter, J. Org. Chem., 26 (1961) 1491 und Verbindung VI/4 wurde nach E. Wünsch und K.H. Deimer, Hoppe-Seyler's Z. Physiol. Chem., 353 (1972) 1246 hergestellt.

Es zeigte sich, daß der Herstellungsweg (über VI/I → VI/4) ungewöhnlich rasch und einfach zum Ziele führte, da die einzelnen Zwischenstufen kristallin, in guten Ausbeuten und hoher Reinheit anfielen. Bemerkenswert einfach ist der Hydrierschritt, der das Dipeptid von den Schutzgruppen befreit, durchzuführen (Beispiel 5.2).

Die verwendeten Abkürzungen bedeuten:

| | |
|---|---|
| Boc | tert.-Butyloxycarbonyl |
| DCCI | Dicyclohexylcarbodiimid |
| DMF | Dimethylformamid |
| Et | Ethyl |
| HOBt | 1-Hydroxybenzotriazol |
| HONSu | N-Hydroxysuccinimid |
| HOOBt | 3-Hydroxy-4-oxo-3,4-dihydro-1,2,3-benzotriazin |
| MEPA | Methyl-ethylphosphinsäureanhydrid |
| ONb | p-Nitrobenzylester |
| ONSu | N-Hydroxysuccinimidester |
| $OBu^t$ | tert.-Butylester |
| PPA | n-Propylphosphonsäureanhydrid |
| $Bu^t$ | tert.-Butylether |
| Tos-OH | p-Toluolsulfonsäure |
| Z | Benzyloxycarbonyl |
| NEM | N-Ethylmorpholin |

Die nachfolgenden Beispiele sollen das erfindungsgemäße Verfahren erläutern, ohne die Erfindung auf die hier stellvertretend genannten Peptide zu beschränken. Ein Teil der Beispiele beschreibt Vergleichsversuche mit Verfahren des Standes der Technik.

**Beispiel 1.1**

**Herstellung von Z-Trp Ser-Tyr-D-Ser($Bu^t$)-Leu-Arg-Pro-NHEt * Tos-OH mit DCCI/HOBt**

29,4 g (0,05 Mol) Z-Trp-Ser-Tyr-OH, 44,7 g (0,05 Mol H-D-Ser($Bu^t$)-Leu-Arg-Pro-NHEt * 2 Tos-OH und 6,75 g 1-Hydroxybenzotriazol werden in 250 ml DMF gelöst und die Lösung auf + 5°C abgekühlt. Der Lösung werden 6,4 ml N-Ethylmorpholin und 11,3 g DCCI zugesetzt und nach beendeter Zugabe wird die Temperatur auf ca. 20°C erhöht. Nach Rühren über Nacht wird vom ausgefallenen Dicyclohexylharnstoff abgesaugt und das Filtrat auf Rückstand eingedampft. Dieser wird in 600 ml Butanol aufgenommen und mit 2 x 150 ml Sodalösung (5 %-ig), 150 ml Kochsalzlösung (13 %-ig), 150 ml $KHSO_4$-Lösung (10 %-ig) und 150 ml Kochsalzlösung (5 %-ig) extrahiert. Die organische Phase wird konzentriert, vom ausgefallenen Kochsalz abfiltriert und die konzentrierte Lösung (200 ml) in 1,5 l Diisopropylether eingerührt. Nach Filtration und Waschen mit 200 ml Diisopropylether wird die Substanz über $P_2O_5$ im Vakuum getrocknet.

Ausbeute: 44,8 g (69 % d.Th.)
$[\alpha]^{22}_D$ : - 31,6° (c = 1, Dimethylacetamid)

**Beispiel 1.2**

**Herstellung von Z-Trp-Ser-Tyr-D-Ser(Bu$^t$)-Leu-Arg-Pro-NHEt * Tos-OH mit DCCI/HOOBt**

Es wird wie im Beispiel 1.1 verfahren, jedoch anstelle von 1-Hydroxybenzotriazol werden 8,15 g 3-Hydroxy-4-oxo-3,4-dihydro-1,2,3-benzotriazin(HOOBt) verwendet.

Ausbeute: 46,7 g (72 % d.Th.)
$[\alpha]^{22}_D$ : - 31° (c = 1, Dimethylacetamid)

**Beispiel 1.3**

**Herstellung von Z-Trp-Ser-Tyr-D-Ser(Bu$^t$)-Leu-Arg-Pro-NHEt * Tos-OH mit DCCI/HONSu**

Es wird wie im Beispiel 1.1 verfahren, jedoch anstelle von 1-Hydroxybenzotriazol werden 5,75 g N-Hydroxysuccinimid (HONSu) verwendet:

Ausbeute: 48,7 g (75 % d.Th.)
$[\alpha]^{22}_D$ : - 32° (c = 1, Dimethylacetamid)

**Beispiel 1.4**

**Herstellung von Z-Trp-Ser-Tyr-D-Ser(Bu$^t$)-Leu-Arg-Pro-NHEt * Tos-OH mit PPA**

In 65 ml DMF werden 6,8 g (11,5 mmol) Z-Trp-Ser-Tyr-OH und 10,3 g (11,5 mmol) H-D-Ser(Bu$^t$)-Leu-Arg-Pro-NHEt* 2 Tos-OH bei Raumtemperatur gelöst und die Lösung auf + 5°C abgekühlt. Aus zwei Tropftrichtern werden dann gleichzeitig innerhalb von 20 min. 11,1 ml N-Ethylmorpholin und 11,1 ml n-Propylphosphonsäureanhydrid (50 Gew.-% in Methylenchlorid) zugetropft. Die Reaktionslösung wird über Nacht gerührt (bei Raumtemp.), im Vakuum auf ca. 20 ml einkonzentriert und in 150 ml n-Butanol aufgenommen. Die Butanollösung wird 2 x mit je 30 ml Sodalösung (5 %-ig), 30 ml Kochsalzlösung (13 %-ig), 150 ml $KHSO_4$-Lösung (10 %-ig) und 150 ml Kochsalzlösung (5 %-ig) extrahiert, im Vakuum aufkonzentriert, vom ausgefallenen Kochsalz abfiltriert und die konzentrierte Lösung in ca. 250 ml Diisopropylether eingetropft. Nach Filtration und Waschen mit 30 - 40 ml Diisopropylether wird die Substanz über $P_2O_5$ im Vakuum getrocknet.

Ausbeute: 13,1 g (88 % d.Th.)
$[\alpha]^{22}_D$ : - 33,7° (c = 1, Dimethylacetamid)

**Beispiel 1.5**

**Herstellung von Z-Trp-Ser-Tyr-D-Ser(Bu$^t$)-Leu-Arg-Pro-NHEt * Tos-OH mit MEPA**

Es wird im wesentlichen wie im Beispiel 1.4 verfahren mit Ausnahme der folgenden Abweichungen: Die zu kuppelnden Peptide werden in je 30 ml DMF und Wasser gelöst; 2,3 g MEPA werden in 5 ml DMF verdünnt und zugetropft; mit ca. 3 ml N-Ethylmorpholin wird ein pH-Wert von pH = 7,5 eingestellt.

Ausbeute: 9,4 g (63 % d.Th.)
$[\alpha]^{22}_D$ : - 30,4° (c = 1, Dimethylacetamid)

**Beispiel 1.6**

**Herstellung von Z-Trp-Ser-Tyr-D-Ser(Bu$^t$)-Leu-Arg-Pro-NHEt * HCl nach dem Azidverfahren**

50 g (83 mmol) Z-Trp-Ser-Tyr-$N_2H_3$ werden in 350 ml DMF gelöst, die Lösung auf - 30°C abgekühlt, während 20 min 45 ml einer 8 N HCl in Dioxan zulaufen lassen, 12,5 ml t-Butylnitrit (in 100 ml Dioxan gelöst) innerhalb von 20 min bei mindestens - 20°C zudosiert, dann bei - 20°C 20 min lang gerührt, auf - 30°C gekühlt und mit 45,7 ml N-Ethylmorpholin versetzt. Dieser so zubereiteten Azidlösung werden 55 g (61 mmol) H-D-Ser(Bu$^t$)-Leu-Arg-Pro-NHEt* 2 Tos-OH, die in 150 ml DMF gelöst und mit 15,2 ml N-Ethylmorpholin versetzt sind, zugegeben. Die Reaktionstemperatur wird auf + 5°C erhöht und die Mischung 20 Std. weitergerührt.

Dann wird die Reaktionslösung zwischen 400 ml n-Butanol und 4 l einer 25 %-igen Kochsalzlösung verteilt. Die wäßrige Phase wird noch dreimal mit je 200 ml n-Butanol und die vereinigten organischen Phasen dreimal mit je 200 ml einer 6,7 %-igen $KHSO_4$ / 3,3 %-igen $K_2SO_4$-Lösung extrahiert. Nach Extraktion mit 200 ml 25

%-iger Kochsalzlösung erfolgt die Extraktion mit 200 ml einer 5 %-igen Bicarbonatlösung. Die Butanolphase wird im Vakuum eingedampft und das noch flüssige Konzentrat in 1 l Essigester unter kräftigem Rühren eingetragen. Der ausgefallene Niederschlag wird abfiltriert, mit 200 ml Essigester gewaschen und im Vakuum getrocknet.

Ausbeute: 51,7 g (73 % d.Th.)
$[\alpha]^{22}_D$ : - 31,8° (c = 1 in Methanol)

**Beispiel 2.1**

**Herstellung von Z-Trp-Ser-Tyr-Gly-Leu-Arg-Pro-Gly-$NH_2$ * Tos-OH mit PPA**

29,4 (0,05 Mol) Z-Trp-Ser-Tyr-OH und 42,08 g (0,05 Mol) H-Gly-Leu-Arg-Pro-Gly-$NH_2$ * 2 Tos-OH werden in 200 ml DMF gelöst, die Lösung auf ca. + 5°C gekühlt und 48,6 ml NEM hinzugefügt. Dann werden innerhalb von 20 - 30 min 47,3 ml PPA (50 %-ig in Methylenchlorid) zugetropft, die Temperatur der Reaktionsmischung auf Raumtemperatur gebracht und über Nacht gerührt. Der Umsatztest erfolgt mittels DC-Kontrolle im Laufmittel Butanol/Eisessig/Wasser (3 : 1 : 1). Nach Aufkonzentrieren der Lösung auf ca. 100 ml wird in 600 ml Butanol aufgenommen und mit 2 x 150 ml Sodalösung (5 %-ig), 150 ml Kochsalzlösung (13 %-ig), 150 ml $KHSO_4$-Lösung (10 %-ig) und 150 ml Kochsalzlösung (5 %-ig) extrahiert. Die organische Phase wird konzentriert, vom ausgefallenen Kochsalz abfiltriert und die konzentrierte Lösung (200 ml) in 1,5 l Diisopropylether eingerührt. Nach Filtration und Waschen mit 200 ml Diisopropylether wird die Substanz über $P_2O_5$ im Vakuum getrocknet.

Ausbeute: 55,2 g (89 % d.Th.)
$[\alpha]^{22}_D$ : - 30,1° (c = 1, Methanol)

**Beispiel 2.2**

**Herstellung von Z-Trp-Ser-Tyr-Gly-Leu-Arg-Pro-Gly-$NH_2$ * Tos-OH nach dem Azidverfahren**

57,7 g (95,8 mmol) Z-Trp-Ser-Tyr-$N_2H_3$ werden in 400 ml DMF gelöst, die Lösung auf - 30°C abgekühlt, während 20 min 43,5 ml einer 8 N HCl in Dioxan zulaufen lassen, 13,9 ml t-Butylnitrit (in 125 ml Dioxan gelöst) innerhalb von 20 min bei mindestens - 20°C zudosiert, dann bei - 20°C 20 min lang gerührt, auf - 30°C gekühlt und mit 50 ml N-Ethylmorpholin versetzt. Der Azidlösung werden nun 70 g (83 mmol) H-GlyLeu-Arg-Pro-Gly-$NH_2$ * 2 Tos-OH, die in 400 ml DMF gelöst und mit 30 ml N-Ethylmorpholin versetzt sind, zugegeben. Die Reaktionstemperatur wird auf + 5°C erhöht und die Mischung 20 Std. weitergerührt.

Dann wird die Reaktionslösung zwischen 400 ml n-Butanol und 5 l einer 25 %-igen Kochsalzlösung verteilt. Die wäßrige Phase wird noch zweimal mit je 200 ml n-Butanol und die vereinigten organischen Phasen zweimal mit je 250 ml einer 8 %-igen Bicarbonatlösung und viermal mit je 150 ml einer 13 %-igen Kochsalzlösung extrahiert. Die Butanolphase wird über Natriumsulfat getrocknet und filtriert. Dann wird die Butanollösung in 6 l Essigester eingerührt, der ausgefallene Niederschlag filtriert, mit 500 ml Essigester gewaschen und im Vakuum getrocknet.

Ausbeute: 73 g (71 % d.Th.)
$[\alpha]^{22}_D$ : - 31,8° (c = 1, Methanol)

**Beispiel 3**

**Herstellung von Z-Trp-Ser-Tyr-Gly-Leu-$OBu^t$ mit PPA**

58,9 g (0,1 Mol) Z-Trp-Ser-Tyr-OH und 41,7 g (0,1 Mol) H-Gly-Leu-$OBu^t$ * Tos-OH werden in 400 ml $CH_2Cl_2$ gelöst und die Lösung auf 0° ... + 5°C abgekühlt. Aus zwei Tropftrichtern werden gleichzeitig 96,5 ml N-Ethylmorpholin und 96,5 ml n-Propylphosphonsäureanhydridlösung (50 Gew.-% in Methylenchlorid) unter Kühlung während 20 - 30 min zugetropft. Dann wird auf Raumtemperatur erwärmt und die Reaktionslösung nach 3 - 20 Std. mit 2 x 150 ml Sodalösung (5 %-ig), 150 ml Kochsalzlösung (13 %-ig), 150 ml $KHSO_4$-Lösung (10 %-ig) und Kochsalzlösung (5 %-ig) extrahiert. Die Methylenchloridphase wird im Vakuum eingedampft und der verbleibende Rückstand aus Essigester/Ligroin kristallisiert. Nach Filtration und Waschen mit 100 ml Ligroin wird die Substanz im Vakuum getrocknet.

Ausbeute: 70 g (86 % d.Th.)
$[\alpha]^{22}_D$ : - 25,8° (c = 1, Methanol)

**Beispiel 4.1**

**Herstellung von Z-Trp-Ser-Tyr-$N_3$**

6 g (10 mmol) Z-Trp-Ser-Tyr-$N_2H_3$ werden in 40 ml DMF gelöst, die Lösung auf - 30°C gekühlt, 52,5 ml (42 mmol) 8 N HCl/Dioxan während 20 min zulaufen lassen, 1,4 ml t-Butylnitrit (in 12 ml Dioxan gelöst) innerhalb von 20 min bei - 20°C zudosiert, dann bei - 20°C 20 min lang gerührt, auf - 30°C abgekühlt und mit 5,3 ml N-Ethylmorpholin versetzt. Die kalte Lösung wird in 650 ml kalten Diethylether eingetragen und der Niederschlag durch Dekantieren vom Überstand befreit, erneut mit kaltem Ether verrührt und dekantiert. Der Rückstand wird in 40 ml Methylenchlorid aufgenommen, dreimal mit ca. 25 g Eiswasser extrahiert und die Lösung über Natriumsulfat getrocknet. Bis zur Aufnahme der IR-Spektren wird die so erhaltene Lösung im Kühlschrank aufbewahrt. Die frische Lösung zeigt im IR eine scharfe Bande bei 4,7 µm, nach ca. 1 h entsteht bei 4,5 µm ein erneutes Signal, das nach 24 Std. größer als das Signal bei 4,7 µm ist. Nach Schwyzer et al., Helv. Chim. Acta, 44 (1961) 1991 ist diese Beobachtung als Curtius'sche Umlagerung eines Säureazids in das entsprechende Isocyanat zu deuten.

**Beispiel 4.2**

**Umsetzung einer gealterten Azidlösung mit H-D-Ser($Bu^t$)-LeuArg-Pro-NHEt * 2 Tos-OH**

Eine nach Beispiel 4.1 hergestellte Lösung wird einen Tag bei Kühlschranktemperatur gelagert und mit einer Lösung von 5 g (5,6 mmol) H-D-Ser($Bu^t$)-Leu-Arg-Pro-NHEt * 2Tos-OH in 15 ml DMF zusammen mit 1 ml N-Ethylmorpholin versetzt und erneut bei + 5°C über Nacht aufbewahrt. Das Produkt wird gemäß Aufarbeitungsverfahren im Beispiel 1.6 isoliert und der Hydrierung in methanolischer Lösung in Gegenwart von Palladium auf Aktivkohle unterworfen. Das nach Hydrierung erhaltene Reaktionsprodukt wurde mittels HPLC (System: Säule: Nucleosil$^{(R)}$ 5SA 4 x 300 mm ; Elutionsmittel: 40 % Puffer/ 60 % Acetonitril (Puffer: 1 %-ig $KH_2PO_4$; PH: 5,8) Fluß: 1 ml/min; Detektor: UV - 220 nm) untersucht.

Dabei trat neben dem Hauptprodukt H-Trp-Ser-Tyr-D-Ser($Bu^t$)-Leu-Arg-Pro-NHEt mit einer Retentionszeit von ca. 25 min ein weiterer intensiver Nebenprodukt-Peak mit einer Retentionszeit von ca. 30 min in Erscheinung. Eine vergleichende Untersuchung der entsprechenden Produkte, die gemäß Beispiel 1.1 - 1.6 hergestellt worden waren, ergab, daß nur das Produkt nach 1.6 den Nebenprodukt-Peak mit einer Retentionszeit von ca. 30 min zu etwa 15 Flächen- % enthielt, während die Produkte nach dem erfindungsgemäßen, neuen Verfahren diese Nebenkomponente nicht aufwiesen.

Offenbar handelt es sich bei dem Nebenprodukt mit der Retentionszeit von ca. 30 min im HPLC um ein Harnstoffderivat, das als Folgeprodukt der im Beispiel 4.1 beobachteten Curtius'schen Umlagerung des Azids in das Isocyanat anzusehen ist.

**Beispiel 5.1**

**Herstellung von Z-Ser-Tyr-ONb**

In einem 4 l-Vierhalskolben werden bei Raumtemperatur in 1,8 l Dimethylformamid, 244,0 g Tyrosin-p-nitrobenzylester Toluolsulfonat und 119,6 g Benzyloxycarbonyl-serin gelöst und die Lösung auf 0 - + 5°C gekühlt. Bei ca. 5°C werden 300,0 ml N-Ethylmorpholin und während 20 min. 275,0 ml ein Propanphosphonsäureanhydrid/Methylenchlorid-Lösung zugetropft.

Danach wird die Temperatur innerhalb einer halben Stunde auf Raumtemperatur erhöht.

Nach ca. 4 Stunden wird mit der Destillation des DMFs unter Vakuum begonnen (am Rotationsverdampfer). Sobald die Destillation beendet ist, wird der Rückstand in Essigester gelöst und Wasser zu einer klaren Lösung verrührt. Der pH-Wert der wäßrigen Phase soll etwa pH 6 betragen. Die Essigesterphase wird im Scheidetrichter abgetrennt, die wäßrige Phase erneut mit Essigester ausgerührt, beide organische Phasen werden vereinigt und die verbleibende wäßrige Phase wird nach DC-Kontrolle verworfen. Die organische Phase wird mit $KHSO_4$-Lösung und $NaHCO_3$-Lösung, Wasser jeweils ausgerührt und abgetrennt. Die Essigesterlösung wird im Vakuum zur trockne eingedampft, der ölige Rückstand wird mit Diethylether verrührt, wobei das öl durchkristallisiert. Das Produkt wird abgesaugt und im Vakuum getrocknet.

Ausbeute: 174,8 g (65 % d.Th.)
Fp: 202 - 208° (Zers.),
$[\alpha]^{22}_D$ : - 13,2° (c = 1, Dimethylacetamid)

**Beispiel 5.2**

**Herstellung von Seryl-tyrosin (H-Ser-Tyr-OH)**

In einem 4 l Vierhalskolben werden bei Raumtemperatur in 2,0 l Methanol und 1,0 l Dimethylformamid 177,0 g Benzyloxycarbonyl-seryl-tyrosin-p-nitrobenzylester (Z-Ser-Tyr-ONb) gelöst. Die Lösung wird mit 10,0 g wasserfeuchtem Katalysator (Pd/Aktivkohle) versetzt und unter Rühren bei Raumtemperatur 2 Std. hydriert.

Es wird dann filtriert, zweimal mit Methanol/DMF (2 : 1) nachgewaschen. Das Filtrat wird im Vakuum zur Trockne eingedampft. Der Rückstand wird in Diisopropylether eingerührt, wobei das Produkt als flockiger, schnell sedimentierender Niederschlag anfällt und abgesaugt werden kann. Das Produkt wird im Vakuum getrocknet.

Ausbeute: 100,3 g (87 % d.Th.)
$[\alpha]^{22}_{D}$ : + 33,2° (c = 1, Methanol)

**Beispiel 5.3**

**Herstellung von Z-Trp-Ser-Tyr-OH**

In 500 ml DMF werden 100,3 g Seryl-tyrosin (Gebalt 77 %) suspendiert. Der Suspension werden 249 g einer 50 %-igen Lösung von Z-Trp-ONSu in DMF und 35 ml N-Ethylmorpholin zugesetzt. Nach ca. 2 Std. hat sich die Suspension unter Rühren bei Raumtemperatur aufgelöst. Das Lösungsmittel wird im Vakuum entfernt und der Rückstand in 500 ml Essigester aufgenommen. Die Essigesterphase wird mit 1 l 85 %-iger Natriumbicarbonatlösung ausgerührt, die organische Phase wird nach DC-Kontrolle verworfen, erneut mit 1 l Essigester überschichtet und mit 1,4 l einer 10 %-igen $KHSO_4$-Lösung vorsichtig sauer gestellt. Die organische Phase wird abgetrennt, mit 1 l Wasser gewaschen und im Vakuum auf ca. 300 ml aufkonzentriert, wobei das Produkt schon auszukristallisieren beginnt. Die Kristallisation wird durch Zugabe von 1,5 l Diisopropylether vervollständigt. Der Niederschlag wird abfiltriert und bei 40°C im Vakuum getrocknet.

Ausbeute: 126,7 g ( 75 % d.Th.)
Fp.: 165 - 172°C;
$[\alpha]^{22}_{D}$ : + 6,2° (c = 1, Methanol)

**Beispiel 6.1**

**Herstellung von Z-Trp-Ser-Tyr-D-Ser(Bu$^t$)-Leu-Arg Pro-NHEt * Tos-OH**

In einem 100 l-Rührbehälter werden 60 kg (63 l) DMF vorgelegt, darin nacheinander 10,34 kg (11,5 Mol) H-D-Ser(Bu$^t$)-Leu-Arg-Pro-NHEt * 2 Tos-OH, 6,77 kg (11,5 Mol) Z-Trp-Ser-Tyr-OH aufgelöst und die Lösung auf + 5°C abgekühlt. In das Zulaufgefäß werden 10,13 kg (11,12 l) N-Ethylmorpholin gefüllt und zunächst davon 1,5 l in die Reaktionslösung einfließen lassen.

Während einer Zeit von ca. 1 Std. werden zwischen + 5 - + 10°C 15,3 kg (11,1 l) n-Propylphosphonsäureanhydrid (50 %-ig in Methylenchlorid) zusammen mit der restlichen N-Ethylmorpholinmenge eingepumpt. Nach beendeter Zugabe wird die Reaktionstemperatur auf etwa 25°C erhöht und der Ansatz 5 Std. gerührt. Die Reaktionslösung wird dann durch Vakuumdestillation auf ca. 25 l Restvolumen aufkonzentriert. Der konzentrierte Rückstand wird mit 150 l n-Butanol in einen 250 l Rührbehälter mit gleich großem Trenngefäß überführt dann wird die Butanolphase nacheinander mit folgenden wäßrigen Lösungen extrahiert:

1) Sodalösung
2) Kochsalzlösung
3) Sodalösung
4) Wasser
5) $KHSO_4$-Lösung und
6) Kochsalzlösung

Die organische Phase wird auf ca. 50 l konzentriert, das Konzentrat in das Zulaufgefäß des Rührbehälters transferiert, der Rührbehälter wird mit 165 kg (230 l) Diisopropylether gefüllt und in diese gerührte Flüssigkeit werden während 1 Std. 25 l des Butanolkonzentrates eindosiert. Der entstandene Niederschlag wird noch 1 Std. nachgerührt und schließlich abzentrifugiert. Mit der zweiten Hälfte des Butanolkonzentrates wird ebenso verfahren. Die Trocknung des Produktes erfolgt im Vakuum bei 35 - 40°C.

Ausbeute: 13,5 kg (90 % d.Th.)
$[\alpha]^{22}_{D}$ : - 33,7° (c = 1, Dimethylacetamid)

**Patentansprüche** für die Vertragsstaate: BE, SE

1. Verfahren zur Herstellung von Peptiden der Formel I

$$U - A^1 - A^2 - A^3 - A^4 - A^5 - X \qquad (I)$$

in welcher
U eine Urethanschutzgruppe,
$A^1$ Trp oder D-Trp,
$A^2$ Ser, Ala oder Thr,
$A^3$ Tyr oder Phe,
$A^4$ Gly, den Rest einer D-Aminosäure oder den Rest eines D-Aminosäurederivates,
$A^5$ Leu, N-Methyl-Leu, N-Ethyl-Leu, Ser($Bu^t$), Cys($Bu^t$), Asp($OBu^t$), Glu($OBu^t$), Orn(Boc) oder Lys(Boc) und
X $OBu^t$ oder $A^6$-Pro-Y bedeuten,
wobei
$A^6$ für Arg, Orn, Lys oder Homoarginin und
Y für Gly-$NH_2$, NH-NH-CO-$NH_2$, ($C_1$-$C_3$)-Alkylamino, Cyclopropylamino, mit Hydroxy oder Fluor substituiertes ($C_1$-$C_3$)-Alkylamino oder mit Hydroxy oder Fluor substituiertes Cycloalkylamino steht, dadurch gekennzeichnet, daß man ein Tripeptid der Formel II

$$U - A^1 - A^2 - A^3 - OH \qquad (II)$$

in welcher U, $A^1$, $A^2$ und $A^3$ die oben genannte Bedeutung haben, mit einem Peptid der Formel III

$$H - A^4 - A^5 - X \qquad (III)$$

in welcher $A^4$, $A^5$ und X die oben genannte Bedeutung haben, oder dessen Derivat umsetzt.

2. Verfahren gemäß Anspruch 1, dadurch gekennzeichnet, daß U Benzyloxycarbonyl (Z) bedeutet.

3. Verfahren gemäß Anspruch 1 oder 2, dadurch gekennzeichnet, daß

$A^1$ Trp,
$A^2$ Ser,
$A^3$ Tyr,
$A^4$ Gly, D-Ala, D-Leu, D-Ser($Bu^t$), D-Trp, D-Phe, D-Gln(cyclohexyl), D-Naphthylalanin, D-Benzylhistidin, D-Thr($Bu^t$), D-Cys($Bu^t$), D-Asp($OBu^t$), D-Glu($OBu^t$), D-Orn(Boc) oder D-Lys(Boc), $A^5$ Leu und
X $OBu^t$, Arg-Pro-$NHC_2H_5$, Arg-Pro-Gly-$NH_2$ oder Arg-Pro-NH-NH-CO-$NH_2$ bedeuten.

4. Verfahren gemäß einem der Ansprüche 1 - 3 zur Herstellung von

U-Trp-Ser-Tyr-Gly-Leu-Arg-Pro-Gly-$NH_2$.

5. Verfahren gemäß einem der Ansprüche 1 - 4 zur Herstellung von

U-Trp-Ser-Tyr-D-Ser($Bu^t$)-Leu-Arg-Pro-$NHC_2H_5$.

6. Verfahren gemäß einem der Ansprüche 1 - 5, dadurch gekennzeichnet, daß es in Gegenwart von Dicyclohexylcarbodiimid unter Zusatz von 1-Hydroxybenzotriazol oder 3-Hydroxy-4-oxo-3,4-dihydro-1,2,3-benzotriazin oder N-Hydroxysuccinimid durchgeführt wird.

7. Verfahren gemäß einem der Ansprüche 1 - 5, dadurch gekennzeichnet, daß es in Gegenwart von Anhydriden der Alkanphosphonsäuren oder der Dialkylphosphinsäuren durchgeführt wird.

8. Verfahren gemäß Anspruch 7, dadurch gekennzeichnet, daß es in Gegenwart von n-Propylphosphonsäureanhydrid oder Methylethylphosphinsäureanhydrid durchgeführt wird.

9. Tripeptid der Formel II

$$U - A^1 - A^2 - A^3 - OH \qquad (II)$$

in welcher U, $A^1$, $A^2$ und $A^3$ wie im Anspruch 1 definiert sind.

10. Z - Trp - Ser - Tyr - OH

11. Verfahren zur Herstellung eines Tripeptids gemäß Anspruch 9 oder 10, dadurch gekennzeichnet, daß es durch in der Peptidchemie übliche Fragmentkondensation von Peptidbruchstücken nach dem Kondensationsschema (1 - 2) + 3 oder 1 + (2 - 3) herstellt, wobei andere funktionelle Gruppen gegebenenfalls durch im alkalischen oder schwach sauren Medium abspaltbare Schutzgruppen gegebenenfalls intermediär blockiert werden.

**Patentansprüche** für den Vertragsstaat : A

1. Verfahren zur Herstellung von Peptiden der Formel I

$$U - A^1 - A^2 - A^3 - A^4 - A^5 - X \qquad (I)$$

in welcher
$A^1$ Trp oder D-Trp,
$A^1$Ser, Ala oder Thr,
$A^3$ Tyr oder Phe,
$A^4$ Gly, den Rest einer D-Aminosäure oder den Rest eines D-Aminosäurederivates,
$A^5$ Leu, N-Methyl-Leu, N-Ethyl-Leu, Ser($Bu^t$), Cys($Bu^t$), Asp($OBu^t$), Glu($OBu^t$), Orn(Boc) oder Lys(Boc) und
X $OBu^t$ oder $A^6$-Pro-Y bedeuten,
wobei
$A^6$ für Arg, Orn, Lys oder Homoarginin und
Y für Gly-$NH_2$, NH-NH-CO-$NH_2$, $(C^1$-$C^3)$-Alkylamino, Cyclopropylamino mit Hydroxy oder Fluor substituiertes $(C_1$-$C_3)$-Alkylamino oder mit hydroxy oder Fluor substituiertes Cycloalkylamin steht, dadurch gekennzeichnet, daß man ein Tripeptid der Formel II

$$U - A^1 - A^2 - A^3 - OH \qquad (II)$$

in welcher U, $A^1$, $A^2$ und $A^3$ die oben genannt Bedeutung haben, mit einem Peptid der Formel III

$$H - A^4 - A^5 - X \qquad (III)$$

in welcher $A^4$, $A^5$ und X die oben genannte Bedeutung haben oder dessen Derivat umsetzt.

2. Verfahren gemäß Anspruch 1, dadurch gekennzeichnet, daß U Benzyloxycarbonyl (Z) bedeutet.

3. Verfahren gemäß Anspruch 1 oder 2, dadurch gekennzeichnet, daß

$A^1$ Trp,
$A^2$ Ser,
$A^3$ Tyr,
$A^4$ Gly, D-Ala-D-Leu, D-Ser($Bu^t$), D-Trp, D-Phe, D-Gln(cyclohexyl), D-Naphthylalanin, D-Glu($OBu^t$), D-Lys(Boc), D-Benzylhistidin, D-Thr($Bu^t$), D-Cys($Bu^t$), D-Asp($OBu^t$), D-Glu($OBu^t$), D-Orn(Boc) oder D-Lys(Boc)
und
X $OBu^t$, Arg-Pro-$NHC_2H_5$, Arg-Pro-Gly-$NH_2$ oder Arg-Pro-NH-NH-CO-$NH_2$ bedeuten.

4. Verfahren gemäß einem der Ansprüche 1 - 3 zur Herstellung von

U-Trp-Ser-Tyr-Gly-Leu-Arg-Pro-Gly-$NH_2$.

5. Verfahren gemäß einem der Ansprüche 1 - 4 zur Herstellung von

U-Trp-Ser-Tyr-D-Ser($Bu^t$)-Leu-Arg-Pro-$NHC_2H_5$.

6. Verfahren gemäß einem der Ansprüche 1 - 5, dadurch gekennzeichnet, daß es in Gegenwart von Dicyclohexylcarbodiimid unter Zusatz von 1-Hydroxybenzotriazol oder 3-Hydroxy-4-oxo-3,4-dihydro-1,2,3-benzotriazin oder N-Hydroxysuccinimid durchgeführt wird.

7. Verfahren gemäß einem der Ansprüche 1 - 5, dadurch gekennzeichnet, daß es in Gegenwart von Anhydriden der Alkanphosphonsäuren oder der Dialkylphosphinsäuren durchgeführt wird.

8. Verfahren gemäß Anspruch 7, dadurch gekennzeichnet, daß es in Gegenwart von n-Propylphosphonsäureanhydrid oder Methylethylphosphonsäureanhydrid durchgeführt wird.

9. Verfahren zur Herstellung eines Tripeptids der Formel II

$$U - A^1 - A^2 - A^3 - OH \qquad (II)$$

in welcher U, $A^1$, $A^2$ und $A^3$ wie im Anspruch 1 definiert sind, dadurch gekennzeichnet, daß es durch in der Peptidchemie übliche Fragmentkondensation von Peptidbruchstücken nach dem Kondensationsschema (1 - 2) + 3 oder 1 + (2 - 3) herstellt, wobei andere funktionelle Gruppen gegebenenfalls durch im alkalischen oder schwach sauren Medium abspaltbare Schutzgruppen gegebenenfalls intermediär blockiert werden.

10. Verfahren gemäß Anspruch 9, dadurch gekennzeichnet, daß Z-Trp-Ser-Tyr-OH hergestellt wird.

**Claims** for the contracting states: BE, CH, LI, DE, FR, GB, IT, LU, NL, SE

1. A process for the preparation of peptides of the formula I

$$U - A^1 - A^2 - A^3 - A^4 - A^5 - X \qquad (I)$$

in which
U denotes a urethane protective group,
$A^1$ denotes Trp or D-Trp,
$A^2$ denotes Ser, Ala or Thr,
$A^3$ denotes Tyr or Phe,
$A^4$ denotes Gly, the residue of a D-amino acid or the residue of a D-amino acid derivative,
$A^5$ denotes Leu, N-methyl-Leu, N-ethyl-Leu, Ser($Bu^t$), Cys($Bu^t$), Asp($OBu^t$), Glu($OBu^t$), Orn(Boc) or Lys(Boc) and
X denotes $OBu^t$ or $A^6$-Pro-Y,
where
$A^6$ represents Arg, Orn, Lys or homoarginine, and
Y represents Gly-$NH^2$, NH-NH-CO-$NH^2$, ($C_1$-$C_3$)-alkylamino, cyclopropylamino, ($C_1$-$C_3$)-alkylamino which is substituted with hydroxyl or fluorine, or cycloalkylamino which is substituted with hydroxyl or fluorine, which process comprises reacting a tripeptide of the formula II

$$U - A^1 - A^2 - A^3 - OH \qquad (II)$$

in which U, $A^1$, $A^2$ and $A^3$ have the abovementioned meaning, with a peptide of the formula III

$$H - A^4 - A^5 - X \qquad (III)$$

in which $A^4$, $A^5$ and X have the abovementioned meaning, or its derivative.

2. The process as claimed in claim 1, wherein U denotes benzyloxycarbonyl (Z).

3. The process as claimed in claim 1 or 2, wherein

$A^1$ denotes Trp,
$A^2$ denotes Ser,
$A^3$ denotes Tyr,
$A^4$ denotes Gly, D-Ala, D-Leu, D-Ser($Bu^t$), D-Trp, D-Phe, D-Gln(cyclohexyl), D-naphthylalanine, D-benzylhistidine, D-Thr($Bu^t$), D-Cys($Bu^t$), D-Asp($OBu^t$), D-Glu($OBu^t$), D-Orn(Boc) or D-Lys(Boc),
$A^5$ denotes Leu and
X denotes $OBu^t$, Arg-Pro-$NHC_2H_5$, Arg-Pro-Gly-$NH_2$ or Arg-Pro-NH-NH-CO-$NH_2$.

4. The process as claimed in one of claims 1 - 3 for the preparation of

U-Trp-Ser-Tyr-Gly-Leu-Arg-Pro-Gly-$NH_2$.

5. The process as claimed in one of claims 1 - 4 for the preparation of

U-Trp-Ser-Tyr-D-Ser($Bu^t$)-Leu-Arg-Pro-$NHC_2H_5$.

6. The process as claimed in one of claims 1 - 5, which is carried out in the presence of dicyclohexylcarbodiimide with the addition of 1-hydroxybenzotriazole or 3-hydroxy-4-oxo-3,4-dihydro-1,2,3-benzotriazine or N-hydroxysuccinimide.

7. The process as claimed in one of claims 1 - 5, which is carried out in the presence of anhydrides of alkanephosphonic acids or of dialkylphosphinic acids.

8. The process as claimed in claim 7, which is carried out in the presence of n-propylphosphonic anhydride or methylethylphosphinic anhydride.

9. A tripeptide of the formula II

$$U - A^1 - A^2 - A^3 - OH \qquad (II)$$

in which U, $A^1$, $A^2$ and $A^3$ are defined as in claim 1.

10. Z - Trp - Ser - Tyr - OH.

11. A process for the preparation of a tripeptide as claimed in claim 9 or 10, which is prepared by fragment condensation, which is customary in peptide chemistry, of peptide fragments according to the condensation scheme (1 - 2) + 3 or 1 + (2 - 3), where appropriate intermediate blocking of other functional groups being carried out, where appropriate, by protective groups which can be eliminated in an alkaline or weakly acid medium.

**Claims** for the contracting state: A

1. A process for the preparation of peptides of the formula I

$U - A^1 - A^2 - A^3 - A^4 - A^5 - X$ (I)

in which
U denotes a urethane protective group,
$A^1$ denotes Trp or D-Trp,
$A^2$ denotes Ser, Ala or Thr,
$A^3$ denotes Tyr or Phe,
$A^4$ denotes Gly, the residue of a D-amino acid or the residue of a D-amino acid derivative,
$A^5$ denotes Leu, N-methyl-Leu, N-ethyl-Leu, $Ser(Bu^t)$, $Cys(Bu^t)$, $Asp(OBu^t)$, $Glu(OBu^t)$, Orn(Boc) or Lys(Boc) and
X denotes $OBu^t$ or $A^6$-Pro-Y,
where
$A^6$ represents Arg, Orn, Lys or homoarginine, and
Y represents Gly-$NH_2$, NH-NH-CO-$NH_2$, ($C_1$-$C_3$)-alkylamino, cyclopropylamino, ($C_1$-$C_3$)-alkylamino which is substituted with hydroxyl or fluorine, or cycloalkylamino which is substituted with hydroxyl or fluorine, which process comprises reacting a tripeptide of the formula II

$U - A^1 - A^2 - A^3 - OH$ (II)

in which U, $A^1$, $A^2$ and $A^3$ have the abovementioned meaning, with a peptide of the formula III

$H - A^4 - A^5 - X$ (III)

in which $A^4$, $A^5$ and X have the abovementioned meaning, or its derivative.

2. The process as claimed in claim 1, wherein U denotes benzyloxycarbonyl (Z).

3. The process as claimed in claim 1 or 2, wherein
$A^1$ denotes Trp,
$A^2$ denotes Ser,
$A^3$ denotes Tyr,
$A^4$ denotes Gly, D-Ala, D-Leu, D-$Ser(Bu^t)$, D-Trp, D-Phe, D-Gln(cyclohexyl), D-naphthylalanine, D-$Glu(OBu^t)$, D-Lys(Boc), D-benzylhistidine, D-$Thr(Bu^t)$, D-$Cys(Bu^t)$, D-$Asp(OBu^t)$, D-$Glu(OBu^t)$, D-Orn(Boc) or D-Lys(Boc), and
X denotes $OBu^t$, Arg-Pro-$NHC_2H_5$, Arg-Pro-Gly-$NH_2$ or Arg-Pro-NH-NH-CO-$NH_2$.

4. The process as claimed in one of claims 1 - 3 for the preparation of

U-Trp-Ser-Tyr-Gly-Leu-Arg-Pro-Gly-$NH_2$.

5. The process as claimed in one of claims 1 - 4 for the preparation of

U-Trp-Ser-Tyr-D-$Ser(Bu^t)$-Leu-Arg-Pro-$NHC_2H_5$.

6. The process as claimed in one of claims 1 - 5, which is carried out in the presence of dicyclohexylcarbodiimide with the addition of 1-hydroxybenzotriazole or 3-hydroxy-4-oxo-3,4-dihydro-1,2,3-benzotriazine or N-hydroxysuccinimide.

7. The process as claimed in one of claims 1 - 5, which is carried out in the presence of anhydrides of alkanephosphonic acids or of dialkylphosphinic acids.

8. The process as claimed in claim 7, which is carried out in the presence of n-propylphosphonic anhydride or methylethylphosphinic anhydride.

9. A process for the preparation of a tripeptide of the formula II

U-$A^1$-$A^2$-$A^3$-OH (II)

in which U, $A^1$, $A^2$ and $A^3$ are defined as in claim 1, which is prepared by fragment condensation, which is customary in peptide chemistry, of peptide fragments according to the condensation scheme (1 - 2) + 3 or 1 + (2 - 3), where appropriate intermediate blocking of other functional groups being carried out, where appropriate, by protective groups which can be eliminated in an alkaline or weakly acid medium.

10. The process as claimed in claim 9, in which Z-Trp-Ser-Tyr-OH is prepared.

**Revendications** pour les États Contractants: BE, CH, LI, DE, FR, GB, IT, LU, NL, SE

1. Procédé de préparation de peptides de formule I:

U - $A^1$ - $A^2$ - $A^3$ - $A^4$ - $A^5$ - X (I)

dans laquelle
U signifie un groupe protecteur d'uréthane,
$A^1$ Trp ou D-Trp,
$A^2$ Ser, Ala, ou Thr,
$A^3$ Tyr ou Phe,
$A^4$ Gly, le reste d'un D-acide aminé ou le reste d'un dérivé de D-acide aminé,
$A^5$ Leu, N-méthyl-Leu, N-éthyl-Leu, Ser($Bu^t$), Cys($Bu^t$), Asp($OBu^t$), Glu($OBu^t$), Orn(Boc) ou Lys(Boc) et
X $OBu^t$ ou $A^6$-Pro-Y,
où
$A^6$ représente Arg, Orn, Lys ou de l'homoarginine et Y représente Gly-$NH_2$, NH-NH-CO-$NH_2$, ($C_1$-$C_3$)-alkylamino, cyclopropylamino, ($C_1$-$C_3$)-alkylamino substitué par un hydroxy ou du fluor, ou un cycloalkylamino substitué par un hydroxy ou du fluor, caractérisé en ce qu'on fait réagir un tripeptide de formule II:

U - $A^1$ - $A^2$ - $A^3$ - OH (II)

dans laquelle U, $A^1$, $A^2$ et $A^3$ ont la signification susmentionnée, avec un peptide de formule III:

H - $A^4$ - $A^5$ - X (III)

dans laquelle $A^4$, $A^5$ et X ont la signification susmentionnée, ou avec un dérivé dudit peptide.

2. Procédé selon la revendication 1, caractérisé en ce que U signifie du benzyloxycarbonyle (Z).

3. Procédé selon l'une quelconque des revendications 1 et 2, caractérisé en ce que

$A^1$ signifie Trp,
$A^2$ Ser,
$A^3$ Tyr,
$A^4$ Gly, D-Ala, D-Leu, D-Ser($Bu^t$), D-Trp, D-Phe, D-Gln(cyclohexyl), D-naphtylalanine, D-benzylhistidine, D-Thr ($Bu^t$), D-Cys($Bu^t$), D-Asp($OBu^t$), D-Glu($OBu^t$), D-Orn(Boc) ou D-Lys(Boc),
$A^5$ Leu et
X $OBu^t$, Arg-Pro-$NHC_2H_5$, Arg-Pro-Gly-$NH_2$ ou Arg-Pro-NH-NH-CO-$NH_2$.

4. Procédé selon l'une quelconque des revendications 1 à 3, pour la préparation de

U-Trp-Ser-Tyr-Gly-Leu-Arg-Pro-Gly-$NH_2$.

5. Procédé selon l'une quelconque des revendications 1 à 4, pour la préparation de

U-Trp-Ser-Tyr-D-Ser($Bu^t$)-Leu-Arg-Pro-$NHC_2H_5$.

6. Procédé selon l'une quelconque des revendications 1 à 5, caractérisé en ce qu'il est réalisé en présence de dicyclohexylcarbodiimide avec addition d'hydroxy-1-benzotriazole ou d'hydroxy-3-oxo-4-dihydro-3,4-benzotriazine-1,2,3 ou de N-hydroxysuccinimide.

7. Procédé selon l'une quelconque des revendications 1 à 5, caractérisé en ce qu'il est réalisé en présence d'anhydrides des acides alcanephosphoniques ou des acides dialkylphosphiniques.

8. Procédé selon la revendication 7, caractérisé en ce qu'il est réalisé en présence d'anhydride d'acide n-pro-pylphos-

phonique ou d'anhydride d'acide méthyléthylphosphinique.

9. Tripeptide de formule II:

$U - A^1 - A^2 - A^3 - OH$ (II)

dans laquelle U, $A^1$, $A^2$ et $A^3$ sont définis comme dans la revendication 1.

10. Z - Trp - Ser - Tyr - OH.

11. Procédé de préparation d'un tripeptide selon l'une quelconque des revendications 9 et 10, caractérisé en ce qu'il est obtenu par condensation fragmentaire, usuelle dans la chimie des peptides, de fragments de peptides selon le schéma de condensation (1 - 2) + 3 ou 1 + (2 - 3), d'autre groupes fonctionnels étant éventuellement bloqués de façon intermédiaire le cas échéant par des groupes protecteurs pouvant être séparés en milieu alcalin ou faiblement acide.

**Revendications** pour l'Etat Contractant: A

1. Procédé de préparation de peptides de formule I:

$U - A^1 - A^2 - A^3 - A^4 - A^5 - X$ (I)

dans laquelle
U signifie un groupe protecteur d'uréthane,
$A^1$ Trp ou D-Trp,
$A^2$ Ser, Ala, ou Thr,
$A^3$ Tyr ou Phe,
$A^4$ Gly, le reste d'un D-acide aminé ou le reste d'un dérivé de D-acide aminé,
$A^5$ Leu, N-méthyl-Leu, N-éthyl-Leu, Ser($Bu^t$), Cys($Bu^t$), Asp($OBu^t$), Glu($OBu^t$), Orn(Boc) ou Lys(Boc) et
X $OBu^t$ ou $A^6$-Pro-Y,
où
$A^6$ représente Arg, Orn, Lys ou de l'homoarginine et
Y représente Gly-$NH_2$, NH-NH-CO-$NH_2$, ($C_1$-$C_3$)-alkylamino, cyclopropylamino, ($C^3$-$C^3$)-alkylamino substitué par un hydroxy ou du fluor, ou un cycloalkylamino substitué par un hydroxy ou du fluor, caractérisé en ce qu'on fait réagir un tripeptide de formule II:

$U - A^1 - A^2 - A^3 - OH$ (II)

dans laquelle U, $A^1$, $A^2$ et $A^3$ ont la signification susmentionnée, avec un peptide de formule III:

$H - A^4 - A^5 - X$ (III)

dans laquelle $A^4$, $A^5$ et X ont la signification susmentionnée, ou avec un dérivé dudit peptide.

2. Procédé selon la revendication 1, caractérisé en ce que U signifie du benzyloxycarbonyle (Z).

3. Procédé selon l'une quelconque des revendications 1 et 2, caractérisé en ce que

$A^1$ signifie Trp,
$A^2$ Ser,
$A^3$ Tyr,
$A^4$ Gly, D-Ala, D-Leu, D-Ser($Bu^t$), D-Trp, D-Phe, D-Gln(cyclohexyl), D-naphtylalanine, D-benzylhistidine, D-Thr ($Bu^t$), D-Cys($Bu^t$), D-Asp($OBu^t$), D-Glu($OBu^t$), D-Orn(Boc) ou D-Lys(Boc),
et
X $OBu^t$, Arg-Pro-$NHC_2H_5$, Arg-Pro-Gly-$NH_2$ ou Arg-Pro-NH-NH-CO-$NH_2$.

4. Procédé selon l'une quelconque des revendications 1 à 3, pour la préparation de

U-Trp-Ser-Tyr-Gly-Leu-Arg-Pro-Gly-$NH_2$.

5. Procédé selon l'une quelconque des revendications 1 à 4, pour la préparation de

U-Trp-Ser-Tyr-D-Ser($Bu^t$)-Leu-Arg-Pro-$NHC_2H_5$.

6. Procédé selon l'une quelconque des revendications 1 à 5, caractérisé en ce qu'il est réalisé en présence de dicyclohexylcarbodiimide avec addition d'hydroxy-1-benzotriazole ou d'hydroxy-3-oxo-4-dihydro-3,4-benzotriazine-1,2,3 ou de N-hydroxysuccinimide.

7. Procédé selon l'une quelconque des revendications 1 à 5, caractérisé en ce qu'il est réalisé en présence d'anhydrides des acides alcanephosphoniques ou des acides dialkylphosphiniques.

8. Procédé selon la revendication 7, caractérisé en ce qu'il est réalisé en présence d'anhydride d'acide n-propylphosphonique ou d'anhydride d'acide méthyléthylphosphonique.

9. Procédé de préparation d'un tripeptide de formule II

$$U - A^1 - A^2 - A^3 - OH \qquad (II)$$

dans laquelle U, $A^1$, $A^2$ et $A^3$ sont définis comme dans la revendication 1, caractérisé en ce qu'il est obtenu par condensation fragmentaire, usuelle dans la chimie des peptides, de fragments de peptides selon le schéma de condensation (1 - 2) + 3 ou 1 + (2 - 3), d'autres groupes fonctionnels étant éventuellement bloqués de façon intermédiaire le cas échéant par des groupes protecteurs pouvant être séparés en milieu alcalin ou faiblement acide.

10. Procédé selon la revendication 9, caractérisé en ce qu'on prépare du Z-Trp-Ser-Tyr-OH.